# EUROPEAN PATENT APPLICATION

(11) **EP 4 799 617 A1**
(43) Date of publication of application: **02.09.2026**
(21) Application number: 24941471.5
(22) Date of filing: 20.11.2024
(51) Int. Cl.: A61K 31/34, A61K 45/06, A61K 31/519, A61P 17/00, A61P 17/14

(54) **PHARMACEUTICAL COMPOSITION HAVING JAK INHIBITORY EFFECT**

(30) Priority: 29.05.2024 CN 202410683255
(71) Applicant: Orxes Chinese Medicine (Shanghai) Co., Ltd., Shanghai 200120 (CN)
(72) Inventor: WANG, Peng, Shanghai 200120 (CN); ZHAO, Yuqing, Benxi, Liaoning 117004 (CN); LI, Tao, Benxi, Liaoning 117004 (CN)
(74) Representative: Haseltine Lake Kempner LLP
(86) International application number: PCT/CN2024/133167
(87) International publication number: WO 2025/246200

(57) **Abstract**

Disclosed is a pharmaceutical composition, which comprises the following components as active ingredients: (A) a JAK inhibitor or a pharmaceutically acceptable salt thereof; and (B) cedrol. Also disclosed is a formulation, which comprises the pharmaceutical composition and one or more pharmaceutically acceptable excipient. Further disclosed is a method for treating skin diseases caused by a JAK pathway, which comprises orally or topically administering the pharmaceutical composition or the formulation. The pharmaceutical composition can synergistically enhance the curative effect on alopecia areata diseases caused by the JAK pathway, while reducing the dose of prescription, thereby improving the curative effect and medication safety of the composition.

## Description

### FIELD OF THE INVENTION

The present invention relates to the fields of chemical compounding techniques and medical technology. In particular, the present invention relates to a pharmaceutical composition for preventing and treating JAK-caused skin diseases (such as alopecia areata diseases), and preparation method and use thereof.

### BACKGROUND OF THE INVENTION

Janus kinases (JAKs) are a class of intracellular, non-receptor tyrosine kinases that play a central role in signal transduction mediated by many cytokine receptors and participate in critical physiological processes such as proliferation, differentiation, apoptosis, and immune regulation of various types of cells in organisms. Dysregulation of the target JAKs is typically accompanied by various skin diseases, such as alopecia areata (AA). Therefore, they have gradually become one of the most attractive targets in the treatment of AA diseases, and specifically inhibiting the target JAKs is a new strategy for the treatment of AA.

Baricitinib (BT), an oral JAK inhibitor capable of inhibiting all types of JAKs, has shown good efficacy in the treatment of AA. In June 2022, the U.S. Food and Drug Administration (FDA) approved BT as being suitable for treating AA, making it the first FDA-approved, systemic treatment for AA. However, BT has certain limitations when used for treating AA. At present, BT is only available as an oral formulation on the market, and its long-term administration may entail a higher risk of systemic immunosuppression side effects.

Furthermore, although JAK inhibitors have shown promising prospects in the treatment of AA, some patients have also developed resistance to these drugs, making the disease difficult to treat. Therefore, scientists have gradually shifted their attention to combination therapies to enhance therapeutic effects.

Currently, there still remains a need to develop a pharmaceutical composition that is more effective for treating skin diseases, especially AA, and involves reduced drug resistance to JAK inhibitors and less side effects.

### SUMMARY OF THE INVENTION

To meet the above need, the present invention provides a pharmaceutical composition having JAK inhibitory effect, which is capable of preventing and/or treating various types of skin diseases caused by the JAK pathway, especially AA diseases.

Thus, the first aspect of the present application provides a pharmaceutical composition, comprising the following components as active ingredients:
(A) a JAK inhibitor or a pharmaceutically acceptable salt thereof; and
(B) cedrol (abbreviated as CE).

The second aspect of the present application provides a formulation, comprising the pharmaceutical composition according to the first aspect of the present application and one or more pharmaceutically acceptable excipients.

The third aspect of the present application provides use of the pharmaceutical composition according to the first aspect of the present application or the formulation according to the second aspect of the present application in preventing and/or treating skin diseases caused by the JAK pathway, especially AA diseases.

The fourth aspect of the present application provides a method for treating skin diseases caused by the JAK pathway, comprising orally or topically administering the pharmaceutical composition according to the first aspect of the present application or the formulation according to the second aspect of the present application.

The fifth aspect of the present application provides use of the pharmaceutical composition in the manufacture of a medicament for treating skin diseases caused by the JAK pathway, especially AA diseases.

In order to treat various types of skin diseases caused by the JAK pathway, especially AA diseases, the present invention discloses a new pharmaceutical composition that can achieve the following effects by scientifically administering a JAK inhibitor and CE in combination:
(1) exerting a synergistic effect on the JAK signaling pathway to enhance the therapeutic efficacy on the AA diseases caused by the JAK pathway and achieve a synergistically enhanced therapeutic effect; and
(2) reducing the prescribed amounts of the respective active ingredients, reducing the toxic side effects and the development of drug resistance, and improving the efficacy and medication safety of the composition.

The composition or formulation provided by the present invention can be administered orally or topically to act against specific targets, and has remarkable efficacy and medication safety, and offers a new treatment option for patients with AA diseases caused by the JAK pathway.

### BRIEF DESCRIPTION OF THE DRAWINGS

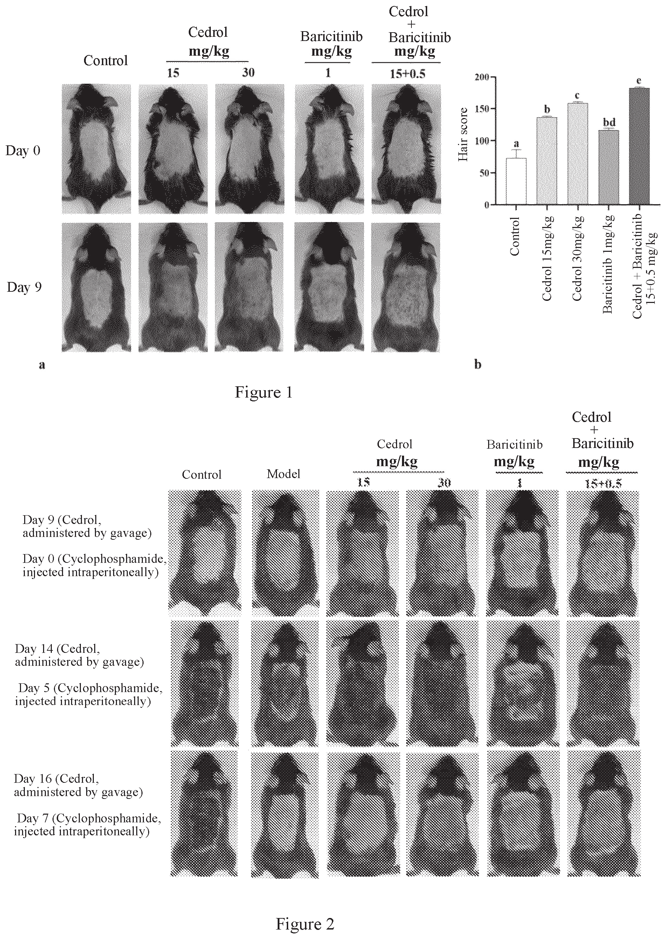

Figure 1 shows the hair growth condition of the mice in Example 1 after 9 days of administration. Figure a shows images taken on the back of the mice on Day 0 and Day 9; and Figure b shows a bar chart of the hair scores of the mice in each group, wherein the hair scores were obtained by analyzing the depilated areas in the images using IMAGE J software (National Institutes of Health, USA).
Figure 2 shows the results of imaging the backs of the mice in each group in Example 1 on Day 9 of administration (Day 0 of CP injection), Day 14 (Day 5 of CP injection), and Day 16 (Day 7 of CP injection).

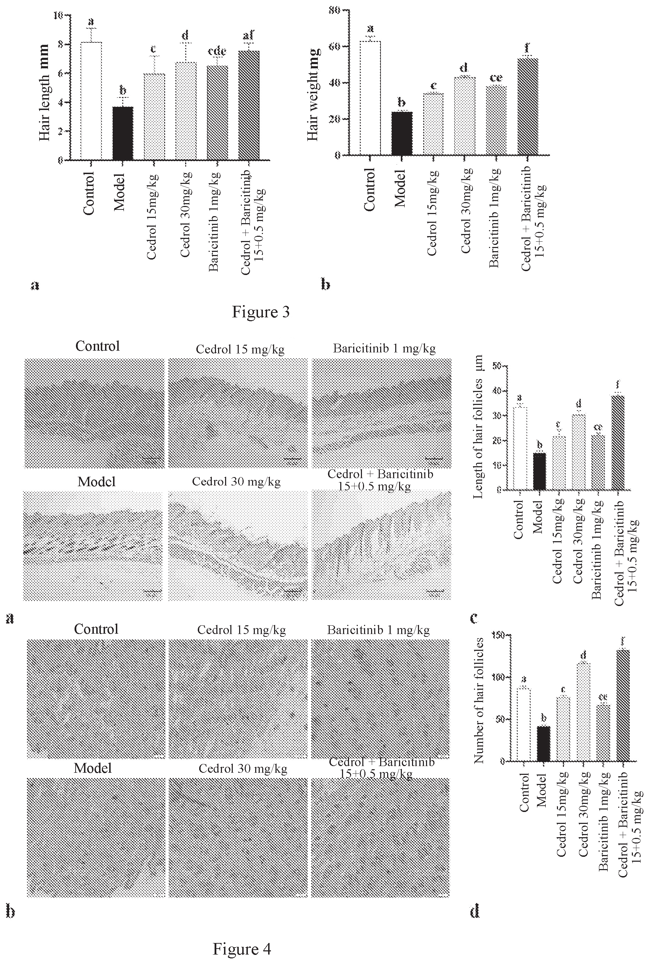

Figure 3 shows the effects of CE, BT and CE+BT on hair length and hair weight in the mice with cyclophosphamide-induced alopecia areata after the final administration, and includes bar charts plotted based on the data of Table 2 obtained in Example 1. Figure a is a bar chart of hair length of the mice in each group after the final administration, and Figure b is a bar chart of hair weight of the mice in each group after the final administration.
Figure 4 shows the effects of CE, BT and CE+BT on the length and number of hair follicles in the skin tissues of the mice with cyclophosphamide-induced alopecia areata. In Figure 4, Figure a shows microscopic images of HE-stained longitudinal sections of skin tissues of the mice in each group for observing the length of hair follicles of mice; Figure b shows microscopic images of HE-stained transverse sections of skin tissues of the mice in each group for observing the number of hair follicles of the mice; Figure c is a bar chart showing the length of hair follicles of the mice in each group as quantified using IMAGE J software; and Figure d is a bar chart showing the number of hair follicles of the mice in each group as quantified using IMAGE J software.

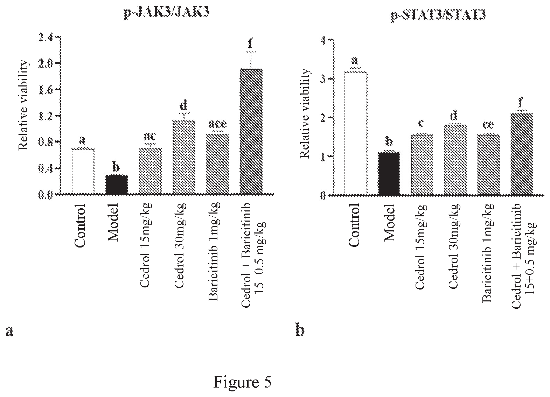

Figure 5 shows the effects of CE, BT and CE+BT on the expression of JAK3/STAT3 protein in the skin tissues of the mice with alopecia areata, and includes bar charts showing the relative viability of the mice in each group. Relative viability is calculated by analyzing the gray values of the bands using IMAGE J software after measuring the protein expressions of p-JAK3, JAK3, p-STAT3, STAT3, and β-actin in the depilated areas of the mice using Western blotting.

In Figures 1 and 2-5, the letters above the bars are marked by using the significance marking method. For details, please refer to the Examples described below.

### DETAILED DESCRIPTION OF THE INVENTION

Some specific embodiments of the present invention will be described in detail below.

JAK inhibitors are immunosuppressants that can selectively inhibit JAK kinases to block the JAK/STAT signaling pathway.

Both JAK inhibitors and cedrol have certain limitations when being used alone to treat various types of skin diseases caused by the JAK pathway, especially AA diseases. For example, baricitinib (BT), one of the JAK inhibitors, is currently available only as an oral formulation on the market, and its long-term administration may carry a higher risk of systemic immunosuppression side effects. Compared with oral formulations, topical formulations can help avoid the first-pass effect in the liver, lower the risk of systemic side effects, and have a prolonged duration of efficacy.

Eedrol (CE), with CAS accession No. 77-53-2, has the following structural formula:

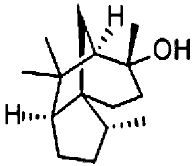

CE is a small-molecule sesquiterpene compound widely found in the volatile oils of various plants in the families of *Cupressaceae, Taxodiaceae, Pinaceae* and the like, and has multiple pharmacological activities, e.g., exerting anti-inflammatory effects or alleviating hair loss.

The inventors of the present application have surprisingly found in practice that the combined use of JAK inhibitors, especially BT, with CE can achieve an unexpected synergistic effect in the treatment of various skin diseases caused by the JAK pathway, especially AA diseases.

Therefore, the present invention provides a pharmaceutical composition, comprising or using the following components as active ingredients:
(A) a JAK inhibitor or a pharmaceutically acceptable salt thereof; and
(B) CE.

In some embodiments, the pharmaceutical composition of the present invention is consisted of Components (A) and (B) as active ingredients.

In some embodiments, the JAK inhibitors include baricitinib (abbreviated as BT, CAS No. 1187594-09-7), ritlecitinib (abbreviated as RT, CAS No. 1792180-81-4), tofacitinib (CAS No. 477600-75-2), oclacitinib (CAS No. 1208319-26-9), decernotinib (CAS No. 944842-54-0), and peficitinib (CAS No. 944118-01-8), filgotinib (CAS No. 1206161-97-8), fedratinib (CAS No. 936091-26-8), lestaurtinib (CAS No. 111358-88-4), pacritinib (CAS No. 937272-79-2), upadacitinib (CAS No. 1310726-60-3), momelotinib (CAS No. 1056634-68-4) and the like, or any combinations thereof.

In some embodiments, said JAK inhibitor is selected from baricitinib (BT), ritlecitinib (RT), tofacitinib, oclacitinib, deuruxolitinib, jaktinib, decernotinib, peficitinib, filgotinib, fedratinib, lestaurtinib, pacritinib, upadacitinib, momelotinib, and any combinations thereof.

The pharmaceutical composition of the present application can comprise one or more JAK inhibitors.

In some preferred embodiments, the JAK inhibitor is BT, RT or a combination thereof.

In some further preferred embodiments, the pharmaceutical composition comprises BT and CE.

In some further preferred embodiments, the pharmaceutical composition comprises RT and CE.

The molar ratio of the JAK inhibitor and CE can be, for example, at least about 1:1000, at least about 1:900, at least about 1:800, at least about 1:700, at least about 1:600, at least about 1:500, at least about 1:400, at least about 1:300, at least about 1:200, at least about 1:100, at least about 1:90, at least about 1:80, at least about 1:70, at least about 1:60, at least about 1:50, at least about 1:40, at least about 1:30, at least about 1:20, at least about 1:10, at least about 1:5, at least about 1:4, at least about 1:3, and at least about 1:1.

The molar ratio of the JAK inhibitor and CE can also be, for example, at most about 1000:1, at most about 900:1, at most about 800:1, at most about 700:1, at most about 600:1, at most about 500:1, at most about 400:1, at most about 300:1, at most about 200:1, at most about 100:1, at most about 90:1, at most about 80:1, at most about 70:1, at most about 60:1, at most about 50:1, at most about 40:1, at most about 30:1, at most about 20:1, at most about 10:1, at most about 5:1, at most about 1:1, at most about 1:2, at most about 1:3, at most about 1:4, at most about 1:5, at most about 1:10, at most about 1:15, at most about 1:20, at most about 1:25, at most about 1:30, at most about 1:35, and at most about 1:40.

Suitable range of the molar ratios of the JAK inhibitor and CE can be any combination of the above ranges. For example, the molar ratio of the JAK inhibitor and CE may be in the range of at least about 1:3 and at most about 3:1, at least about 1:1 and at most about 3:1, or at least about 1:3 and at most about 1:1.

Considering the toxicity of JAK inhibitors, the molar ratio of the JAK inhibitor and CE is preferably less than 1:1, more preferably less than 1:2, even more preferably less than 1:3, even more preferably less than 1:4, even more preferably less than 1:5, even more preferably less than 1:6, even more preferably less than 1:7, even more preferably less than 1:8, even more preferably less than 1:9, and even more preferably less than 1:10.

In some preferred embodiments, the JAK inhibitor is BT, and the molar ratio of BT and CE is 1:(1-100), preferably 1:(20-80), more preferably 1:(30-70), and most preferably 1:(40-60).

The compositions of the present invention can be used in combination with other drugs. Therefore, in some embodiments, the pharmaceutical composition further comprises one or more additional active ingredients that can be used to prevent or treat skin diseases, especially those useful for hair growth, such as minoxidil (CAS No.: 38304-91-5) and finasteride (CAS No.: 98319-26-7).

The pharmaceutical composition of the present invention can be formulated with pharmaceutically acceptable carriers or excipients into clinically acceptable oral or topical formulations. Therefore, the present invention further provides a formulation, comprising the pharmaceutical composition according to the present application and one or more pharmaceutically acceptable carriers or excipients.

In some preferred embodiments, the formulation of the present invention is an oral formulation, for example, a tablet, a pill, a powder, a granule, a capsule, or a lozenge, and other solid formulations.

In some preferred embodiments, the formulation of the present invention is a topical formulation, for example, a glycerite, a tincture, a liniment, a coating agent, an ointment, a cream, a patch, a microneedle, a gel, a liposome, an ethosome or a nanoparticle, and the like.

All the formulations involved in the present application have the conventional meanings known in the art and can be prepared using conventional technical means in the art.

In some preferred embodiments, the pharmaceutical composition in the formulation of the present invention comprises BT and CE, and the formulation is a solid formulation suitable for oral administration.

In some preferred embodiments, the pharmaceutical composition in the formulation of the present invention comprises RT and CE, and the formulation is a solid formulation suitable for oral administration.

In some preferred embodiments, the pharmaceutical composition in the formulation of the present invention comprises BT and CE, and the formulation is a topical formulation.

In some preferred embodiments, the pharmaceutical composition in the formulation of the present invention comprises RT and CE, and the formulation is a topical formulation.

The present invention further provides use of the pharmaceutical composition and the formulation in the prevention and/or treatment of skin diseases caused by the JAK pathway, especially AA diseases.

The present invention further provides a method for preventing and/or treating skin diseases caused by the JAK pathway, which comprises orally administering the pharmaceutical composition or the formulation of the present application.

The present invention further provides a method for preventing and/or treating skin diseases caused by the JAK pathway, which comprises topically administering the pharmaceutical composition or the formulation of the present application.

Considering the toxicity of some JAK inhibitors, the pharmaceutical composition of the present application is preferably administered topically or formulated into a topical formulation.

In the present application, a topical formulation refers to a preparation that can be applied to skin surface.

The present application additionally further provides use of the pharmaceutical composition in the manufacture of a medicament, especially an oral or topical medicament, for treating skin diseases caused by the JAK pathway, especially AA diseases.

The present invention is further described with reference to the following examples, but is not limited thereto.

### EXAMPLES

Information on the key materials used in the examples is described as follows:
CE, an abbreviation of cedrol, was purchased from Zhejing Hangzhou Grascent Co., Ltd., Cat No.: P10-0198;
BT, an abbreviation of baricitinib, purchased from Jiangsu Aikon Biopharmaceutical R&D Co., Ltd., Cat No.: K900933;
CP, an abbreviation of cyclophosphamide, bearing National Drug Approval No. HJ20160467, and having a strength of 0.2 g, was purchased from Baxter Oncology GmbH, 0.2 g strength.

### EXAMPLE 1

### Effects of administering CE and BT in combination on hair growth of mice (C57/BL-6 mice)

60 male C57/BL-6 mice aged 6-8 weeks were evenly divided into 6 groups with 10 mice per group. These groups were: a control group, a model group, a CE (15 mg/kg) group, a CE (30 mg/kg) group, a BT (1 mg/kg) group, and a CE+BT (15+0.5 mg/kg) group. After 1 week of normal feeding, the dorsal hair of the mice was shaved using a depilatory device to prepare the skin (2×3 cm²). Then, depilatory cream was applied to the prepared skin area to ensure complete removal of the hair on the back of the mice. From the day after depilation, each group of mice was administered once daily by gavage. The substances, concentrations, and dosages administered to the mice in each group were shown in Table 1.

**Table 1**

| **Groups** | **Substance to be administered** | **Concentration of the substance to be administered** | **Daily dosage** | **Daily dosage volume** |
|---|---|---|---|---|
| Control group | Aqueous solution of 0.5% sodium carboxymethylcellulose | 100% | 10 ml/kg mouse | |
| Model group | | | | |
| CE (15 mg/kg) | CE in an aqueous solution of 0.5% sodium carboxymethylcellulose | 1.5 mg/mL | 15 mg CE/kg mouse | |
| CE (30 mg/kg) | CE in an aqueous solution of sodium carboxymethylcellulose 0.5% | 3 mg/mL | 30 mg CE/kg mouse | 0.2 mL/20 g mouse |
| BT (1 mg/kg) | BT in an aqueous solution of sodium carboxymethylcellulose 0.5% | 0.1 mg/mL | 1 mg BT/kg mouse | |
| CE+BT (15+0.5 mg/kg) | CE and BT together in an aqueous solution of 0.5% sodium carboxymethylcellulose | CE: 1.5 mg/mL | 15 mg CE+0.5 mg BT /kg mouse | |
| | | BT: 0.05 mg/mL | | |

The substances to be administered were prepared as follows:
**The aqueous solution of 0.5% sodium carboxymethylcellulose:** First, 0.5 g of sodium carboxymethylcellulose powder was added to 100 mL of distilled water, and then the mixture was placed in a water bath and heated at 60-70 °C. During the heating process, the mixture was constantly stirred and supplemented with distilled water to 100 mL. After heating for 5 hours, a 0.5% aqueous solution of sodium carboxymethylcellulose was obtained.

**CE in 0.5% aqueous solution of sodium carboxymethylcellulose, BT in 0.5% aqueous solution of sodium carboxymethylcellulose, and a combination of CE and BT in 0.5% aqueous solution of sodium carboxymethylcellulose:** Appropriate amounts of CE, BT and a CE+BT mixture were weighed and transferred into separate glass mortars, then ground into fine powders using a pestle. Subsequently, 0.5% aqueous solution of sodium carboxymethyl cellulose was gradually added to each mortar under continuous stirring to dissolve the powders. The addition was stopped when no visible particle found in the solution. The administration solutions were prepared as shown in Table 1, which also provides their specific concentrations.

When preparing the solution to be administered in the CE+BT (15+0.5mg/kg) group, CE and BT were combined in the same solution and thereby administered simultaneously during the administration.

Images of the dorsal region of the mice were captured at key time points of hair growth (i.e. Day 0 and Day 9), respectively. The captured images are shown in Figure a of Figure 1. In addition, the gray values in the depilated areas on the dorsal region of the mice in Figure a were analyzed using IMAGE J software (National Institutes of Health, USA) to obtain hair scores. In Figure b, the hair scores represent the quantified gray values of the hair on the back skin of the mice.

The experimental results showed that in the first 9 days after depilation, the mice in the CE (15mg/kg) group, the CE (30mg/kg) group, and the CE+BT (15+0.5mg/kg) group had significant hair growth. The CE+BT (15+0.5mg/kg) group showed the best hair growth, with a significant difference over the CE group.

### Preventive effects of CE, BT and CE+BT on hair loss of the mice with cyclophosphamide-induced alopecia areata

On Day 9 of administration in the aforementioned experiment, cyclophosphamide (abbreviated as CP) was intraperitoneally injected to each mouse in the model group (not administered with CE and/or BT), the CE (15 mg/kg) group, the CE (30 mg/kg) group, the BT (1 mg/kg) group, and the CE+BT (15+0.5 mg/kg) group to establish the AA model. The method for establishing the model is described in the literature: "Effect of CeO2 Nanoenzyme on Hair Growth of Mice with Cyclophosphamide-Induced Alopecia Areata", PENG Xinxin et al., J Shanxi Med Univ, Feb. 2024, Vol. 55, No. 2, p.192.

Whether the model has been successfully established was determined by observing the hair loss in each group of mice, wherein the onset of hair loss indicated the successful model establishment.

The mice in the control group were injected with normal saline on Day 9 of administration. Since normal saline would not cause hair loss, the mice had normal hair growth.

Images of the dorsal region of the mice were captured on Day 9 of administration (Day 0 after CP injection), Day 14 (Day 5after CP injection), and Day 16 (Day 7 after CP injection). The results were shown in Figure 2. In Figure 2, gavage administration was used for administering CE and/or BT to the mice in the CE (15 mg/kg) group, the CE (30 mg/kg) group, the BT (1 mg/kg) group, and the CE+BT (15+0.5 mg/kg) group; and intraperitoneal injection was used for administering CP to the mice in the model group, the CE (15 mg/kg) group, the CE (30 mg/kg) group, the BT (1 mg/kg) group, and the CE+BT (15+0.5 mg/kg) group.

According to the above model, the mice developed dorsal hair loss within one week after CP injection.

As shown in Figure 2, no hair loss was observed in the mice on Day 5 after CP injection, indicating that CP had not yet taken effect. On Day 7 after CP injection, the hair that had regrown in the original depilated area of the mice in the model group started to fall again; that is, the mice had cyclophosphamide-induced AA. Although hair loss was also observed in the mice in the CE (15mg/kg) group, the CE (30mg/kg) group, and the CE+BT (15+0.5mg/kg) group, the condition was significantly better than that of the model group. Clearly, CE and CE+BT had certain preventive effect on hair loss of the mice. Figure 2 also showed that the CE+BT group exhibited the best anti-hair loss effect, and had significant difference over the CE group.

### Effects of CE, BT and CE+BT on hair length and hair weight in the mice with cyclophosphamide-induced alopecia areata

After the final administration (on Day 28), the length of dorsal hair of the mice in each group was measured using a vernier caliper. Subsequently, the regrown hair in the original depilated area was removed using a depilatory device, and was weighed using a balance. The results were shown in Table 2 below.

**Table 2 Effects of CE, BT and CE+BT on hair length and hair weight in the mice with cyclophosphamide-induced alopecia areata**

| Groups | Hair length (mm) | Hair weight (mg) |
|---|---|---|
| Control | 8.35±1.45 | 61.76±4.27 |
| Model | 3.70±0.65 | 24.78±4.50 |
| CE (15 mg/kg) | 5.98±1.22 | 35.38±6.88 |
| CE (30 mg/kg) | 6.78±1.32 | 43.51±6.45 |
| BT (1 mg/kg) | 6.51±0.61 | 38.62±6.02 |
| CE+BT (15+0.5 mg/kg) | 7.58±0.52 | 53.92±4.83 |

Figure 3 includes bar charts plotted based on the data listed in Table 2. Figure a is a bar chart showing hair lengths of the mice in each group after the final administration. Figure b is a bar chart showing hair weights of the mice in each group after the final administration. Through these two figures, the results of hair lengths and hair weights of the mice in each group could be more intuitively compared.

As shown in Table 2 and Figure 3, the mice in the control group exhibit normal hair growth without undergoing the AA process; therefore, they showed the best results in terms of hair length and weight. The mice in the model group only underwent the alopecia areata process without being administered with CE or BT, and thus they exhibit the shortest hair length and the lowest hair weight. The hair length and weight in the CE (15 mg/kg), CE (30 mg/kg) and BT (1 mg/kg) groups were significantly different from and markedly better than those in the model group. Furthermore, in the CE+BT (15 + 0.5 mg/kg) group, the administered dose of CE was half of that in the CE (30 mg/kg) group, and the administered dose of BT was half of that in the BT (1 mg/kg) group; nevertheless, the hair length and hair weight in the combination group were significantly superior to those in either of the two single-agent groups. This indicates that the combination administration of BT and CE by using a composition thereof significantly enhances the efficacy of promoting hair regrowth at a reduced dosage, exhibiting a synergistic effect, and can be used for treating AA. Although BT is a first-line drug for treating AA, it has certain *in vivo* toxicity. Reducing the BT dosage can lower the toxicity and side effects, thereby improving drug safety.

### EXAMPLE 2

### Effects of CE+BT combination on hair follicle length and hair follicle number in mouse skin tissue

The grouping method of mice and administration regimen were the same as those described in Example 1. After the final administration, blood samples were collected from the mice via retro-orbital blood collection method, and then the mice were killed via cervical dislocation. The dorsal hair of the mice was removed using a small animal shaver and collected. The dorsal skin of the mice was lifted using surgical forceps, and surgical scissors were used to cut along the original depilated area on the mouse's back. The excised dorsal skin was further cut along the midline and would be used for different purposes, respectively. One half of the cut skin was placed in 4% paraformaldehyde solution for subsequent HE staining to observe the mouse hair follicle length and number, while the other half was stored in the freezer at - 80°C for Western blotting experiments to detect the expression of p-JAK3/JAK3 and p-STAT3/STA3 proteins.

The half of skin that had been fixed in 4% paraformaldehyde was taken out, embedded, and cut into paraffin sections. The sections were then stained following the HE staining protocol.

The specific staining steps were as follows:
The skin sections of the mice were sequentially immersed in eco-friendly dewaxing solution I for 20 minutes, in eco-friendly dewaxing solution II for 20 minutes, in anhydrous ethanol I for 5 minutes, in anhydrous ethanol II for 5 minutes, and in 75% ethanol for 5 minutes, followed by rinsing with tap water. Subsequently, HE staining was performed using a hematoxylin-eosin (H&E) high-resolution constant staining kit according to the instruction manual. Specifically, the sections were placed in high-resolution constant staining pretreatment solution for 1 minute; afterwards, the sections were immersed in hematoxylin staining solution for 3 to 5 minutes, rinsed with tap water, differentiated with differentiation solution, further rinsed with tap water, blued with the bluing solution, and rinsed with running water. The sections were dehydrated in 95% ethanol for 1 minute, and then stained in eosin staining solution for 15 seconds. Next, the sections were sequentially immersed in anhydrous ethanol I for 2 minutes, anhydrous ethanol II for 2 minutes, anhydrous ethanol III for 2 minutes, n-butanol I for 2 minutes, n-butanol II for 2 minutes, xylene I for 2 minutes, and xylene II for 2 minutes, and then mounted with neutral resin. Microscopic examination was performed on the sections to acquire images and analyze the same. The results were shown in Figure 4.

Some instruments and reagents used in the tests were listed as follows:
**Upright optical microscope:** manufacturer: Nikon, Japan; Cat No.: NIKON ECLIPSE E100;
**Imaging system:** manufacturer: Nikon, Japan; Cat No.: NIKON DS-U3;
**Environmentally friendly dewaxing solution:** manufacturer: Wuhan Servicebio Technology Co., Ltd. (hereinafter referred to as "Servicebio"); Cat No.: G1128;
**General-purpose tissue fixative solution:** manufacturer: Servicebio; Cat No.: G1101; and
**Hematoxylin-Eosin (H&E) High-Resolution Constant Staining Kit:** Manufacturer: Servicebio; Cat No.: G1076.

Figure 4 shows the effects of CE, BT and CE+BT on the length and number of hair follicles in the skin tissues of mice having cyclophosphamide-induced alopecia areata.

In Figure 4, Figure a shows microscopic images of HE-stained longitudinal sections of skin tissues of the mice for observing the length of hair follicles of the mice. Figure b shows microscopic images of HE-stained horizontal sections of skin tissues of the mice for observing the number of hair follicles of the mice. Figure c is a bar chart showing the length of hair follicles of the mice in each group as quantified using IMAGE J software. Figure d is a bar chart showing the number of hair follicles of the mice in each group as quantified using IMAGE J software.

The significance of hair length and weight of the mice among groups was analyzed using the letter-marking method for significant differences. Specifically, in this method, each group was first assigned a distinct letter selected from a to f, respectively; for example, the control group was assigned letter "a", the model group was assigned letter "b", the CE (15mg/kg) group was assigned letter "c", the CE (30mg/kg) group was assigned letter "d", the BT (1mg/kg) group was assigned letter "e", and the CE+BT (15+0.5mg/kg) group was assigned letter "f". When the difference between two groups was not statistically significant (p > 0.05), the same letter (i.e., the previously assigned letter) was marked above the bars for these groups. For example, if no significant difference was observed between the control group and the model group, then the model group's bar would be marked with both its own letter b and the letter a of the group with which it did not significantly differ. Similarly, if no significant difference was observed between the control group and the CE+BT (15+0.5 mg/kg) group, then the CE+BT (15+0.5mg/kg) group's bar was marked with its own letter f and the letter a of the control group with which it did not significantly differ. The significance marking method applied in the bar charts in Figures 1 and 3-5 are performed as described above.

As can be seen from the above results, the CE+BT combination group, administered at half doses, resulted in greater hair follicle length and number in the HE-stained skin of the mice. This group achieved better effects than either the CE group or the BT group, and also had better effects than the control group (which did not undergo the development of alopecia areata), thereby indicating that the combined use of CE and BT resulted in a synergistic effect.

### EXAMPLE 3

### Effects of CE+BT combination on the JAK3/STAT3 signaling pathway in mouse skin tissue

The expression levels of p-JAK3/JAK3 and p-STAT3/STAT3 proteins were detected by Western blotting using the half of the skin samples that were stored in the freezer at -80°C as described in Example 2.
p-JAK3: purchased from Absin, Cat No.: abs139988;
JAK3: purchased from Absin, Cat No.: abs115170;
p-STAT3: purchased from Absin, Cat No.: abs130919;
STAT3: purchased from Absin, Cat No.: abs155916.

The four antibodies mentioned above are referred to as "primary antibodies" in the following tests.

Specific testing methods are briefly described as follows:

### Preparation of supernatant

The mouse skin samples stored at -80°C were taken out. 50 mg of skin tissue was cut with surgical scissors from each sample and respectively placed into 2 mL EP tubes. 500 µL of RIPA lysis buffer (available from Servicebio, Cat No.: G2002-100ML) (containing 5µL of protease inhibitor PMSF, available from Servicebio, Cat No.: G2008-1ML) was added to each EP tube. The skin was then disrupted by a cell grinding rod that is inserted into the 2 mL EP tube. The tube was then left to stand at 4°C for 15 minutes, followed by centrifugation (12,000 r/min, 15 minutes). After centrifugation, the supernatant was collected and used for subsequent total protein concentration assay and protein denaturation.

### Total protein concentration assay

The total protein concentration in the supernatant was determined using a BCA kit (G2026-200T, Servicebio) following the protocol in the instruction manual.

### Protein denaturation

The supernatant was mixed with 5X reducing protein loading buffer (available from Servicebio, Cat No.: G2075-100ML) at a ratio of 4:1. The mixed solution was denatured in a 95°C water bath for 10 minutes, and directly subjected to the subsequent protein electrophoresis steps after cooling.

### Protein electrophoresis

The glass plate was cleaned before gel casting. First, the retaining clips on both sides of the gel casting apparatus were shifted to the bottom position, and the side pressing frames were fully opened. The concave glass plates and flat glass plates, in sequence, were inserted obliquely from the above, then placed down to the bottom until the upper ends of the glass plates being engaged in the clamping grooves on both sides. The side pressing frames were fold back. The left portions of the pressing frames were squeezed simultaneously with hands, and the left retaining clip was fastened upwards to the uppermost position; and then the right potions of the pressing frames were squeezed simultaneously, and the right retaining clip was fastened upwards to the uppermost position. After confirming that the electrophoresis glass plates were tightly clamped and aligned with each other, the knobs on both sides of the gel-casting base were loosened. The electrophoresis support in the central slot of the gel-casting base was placed and locked in place. Subsequently, the main support in place was hold with hands and the knobs were tightened on both sides until they were rotated to the maximum limit.

### Preparation of resolving gel and stacking gel

The resolving gel (10%) and stacking gel (10%) for electrophoresis experiments were prepared using the One-step PAGE Colored Gel Ultra-Rapid Preparation Kit (Cat No.: G2177-50T, available from Servicebio), following the preparation protocol in the kit's instruction manual. The resolving gel and stacking gel were prepared in quadruplicate.

### Electrophoresis

The previously prepared resolving gel and stacking gel were clamped onto the main body of the gel-casting apparatus, with the stacking gel on the top and the resolving gel on the bottom. They were placed in the electrophoresis tank. Subsequently, the inner chamber was filled completely with electrophoresis buffer, and then the outer chamber was filled with the electrophoresis buffer to one-third of its volume. The denatured protein sample obtained in the protein-denaturating step was loaded into the wells in the stacking gel using a pipette. Electrophoresis was performed at a constant voltage of 200V for 30 minutes, and was terminated when the blue indicator dye of the 5X reducing protein loading buffer (the buffer used in the protein-denaturating step) reached about 1 cm above the bottom edge of the gel. The electrophoresis tank was removed. The gel casting apparatus was then disassembled and placed in the transfer buffer (Cat No.: G2028-1L, available from Servicebio) to be ready for subsequent membrane transfer procedure.

### Membrane transfer

Prepare 24 pieces of 7×9 cm transfer filter papers (thin) and 4 pieces of 5×8 cm PVDF membranes. PVDF membranes were activated with ethanol for 2 minutes before use. One transfer clamp, two sponges, filter papers, and activated PVDF membranes were placed into a container containing the transfer buffer (the same transfer buffer with that used in the preceding step where the gel casting apparatus was placed). The transfer clamp was fully opened, with the red part being on the left side and the black part on the right side. One sponge and three pieces of filter paper were placed on each side in sequence. The resolving gel was carefully peeled from the gel casting apparatus and placed on the filter paper (the gel was placed on the black side of the transfer clamp). The gel was rinsed with the transfer buffer to remove air bubbles on its surface. The PVDF membrane was slowly placed onto the resolving gel with great care to avoid air bubbles. Then the transfer filter paper and sponge were covered sequentially. The membrane transfer was performed to transfer the sample from the resolving gel to the PVDF membrane. The membrane transfer conditions (wet transfer) are set as a constant current of 300mA for 30 minutes. The above procedure was repeated four times, to obtain four pieces of PVDF membranes bound with the protein samples.

### Blocking

The sample-bound PVDF membranes obtained from the membrane transfer step were respectively placed into four 1-cell whole-membrane antibody incubation boxes (Cat No.: G9055-1, available from Servicebio) containing TBST (Cat No.: G2150-1L, available from Servicebio), and rinsed rapidly once. Then, 5 ml of 5% skim milk (5 mg of skim milk powder dissolved in 100 ml of water) was added. The membranes were placed on a decoloring shaker and blocked at room temperature for 30 minutes.

### Primary antibody incubation

According to the instruction manuals of the p-JAK3, JAK3, p-STAT3, and STAT3 antibodies (which are primary antibodies), each of the antibodies was diluted separately. The blocking solution in the antibody incubation box was discarded. The diluted primary antibody was added, and incubated overnight at 4°C on a shaker (under gentle shaking).

### Membrane washing

The solution in the antibody incubation box was discarded, and then the membrane was immediately rinsed with TBST three times (1 min, 3 times). TBST was then added, and placed on a decoloring shaker for rapid elution, and washed for 5 minutes per time for three times.

### Secondary antibody incubation

The secondary antibody (HRP-labeled goat anti-rabbit IgG, Cat No: GB23303, available from Servicebio) was diluted with TBST at a volume ratio of 1:5000, and then added to the antibody incubation box mentioned above. The box was placed on a shaker under gentle shaking and incubated at room temperature for 30 minutes.

### Membrane washing

After incubation, the secondary antibody solution in the antibody incubation box was discarded, and then the membrane was rapidly rinsed three times with TBST, followed by three rapid washes with TBST on a decoloring shaker, 5 minutes each. The washed membranes were then subjected to the subsequent chemiluminescence imaging step.

### Imaging and data analysis

Chemiluminescence imaging was performed using a chemiluminescence imaging system (manufacturer: Wuhan Servicebio Technology Co., Ltd., Cat No.: SGG-W2000). After exposure, the original image was saved in TIFF format. Data analyzing was performed on the saved original TIFF image using WB analysis software (manufacturer: Wuhan Servicebio Technology Co., Ltd., Cat No.: AIWBwell^{™}) to obtain protein bands. Then, the IMAGE j software was used to analyze the gray values of the bands to calculate the relative viabilities of p-JAK3/JAK3 and p-STAT3/STAT3. The results were shown in Figure 5.

Figure 5 shows the effects of CE, BT and CE+BT on the expression level of JAK3/STAT3 protein in the skin tissues of the mice with alopecia areata.

As can be seen from Figure 5, first, the relative viabilities of the mice in the CE (15 mg/kg) group and the CE (30 mg/kg) group were significantly different from that in the model group. Second, when BT and CE were administered in combination, with the dosage of each reduced by half, a further enhancement in the relative vitality of the mice was observed. Clearly, the combination of BT and CE exhibits a synergistic effect and can be used for treating alopecia areata.

### Conclusion

In the prevention and treatment of alopecia areata, or diseases caused by the JAK pathway, the combination of CE and a JAK inhibitor shows superior intervention effects compared with CE or the JAK inhibitor alone, exerting a synergistic effect on hair length, hair weight and JAK protein expression.

## Claims

1. A pharmaceutical composition, comprising the following components as active ingredients:
(A) a JAK inhibitor or a pharmaceutically acceptable salt thereof; and
(B) cedrol.

2. The pharmaceutical composition according to claim 1, wherein the JAK inhibitor comprises baricitinib, ritlecitinib, tofacitinib, oclacitinib, deuruxolitinib, jaktinib, decernotinib, peficitinib, filgotinib, fedratinib, lestaurtinib, pacritinib, upadacitinib or momelotinib, and any combinations thereof.

3. The pharmaceutical composition according to claim 1, wherein the JAK inhibitor comprises baricitinib or ritlecitinib, or a combination thereof.

4. The pharmaceutical composition according to claim 1, wherein the pharmaceutical composition comprises baricitinib and cedrol.

5. The pharmaceutical composition according to claim 1, wherein the pharmaceutical composition comprises ritlecitinib and cedrol.

6. The pharmaceutical composition according to claim 1, wherein the JAK inhibitor is baricitinib; and the molar ratio of baricitinib and cedrol is 1: (1-100), more preferably 1: (30-70), and most preferably 1: (40-60).

7. The pharmaceutical composition according to claim 1, wherein the pharmaceutical composition further comprises one or more additional active ingredients that are suitable for preventing or treating skin diseases, especially for hair regrowth.

8. A formulation, comprising the pharmaceutical composition according to any one of claims 1-7 and one or more pharmaceutically acceptable carriers or excipients.

9. The formulation according to claim 8, wherein the formulation is an oral formulation, which is a tablet, a pill, a powder, a granule, a capsule or a lozenge.

10. The formulation according to claim 8, wherein the formulation is a topical formulation, which is a glycerite, a tincture, a liniment, a coating agent, an ointment, a cream, a patch, a microneedle, a gel, a liposome, an ethosome or a nanoparticle.

11. Use of the pharmaceutical composition according to any one of claims 1-7 or the formulation according to any one of claims 8-10 in preventing and/or treating skin diseases caused by the JAK pathway, especially alopecia areata diseases.

12. A method for treating skin diseases caused by the JAK pathway, especially alopecia areata diseases, comprising orally or topically administering the pharmaceutical composition according to any one of claims 1-7 or the formulation according to any one of claims 8-10.

13. Use of the pharmaceutical composition according to any one of claims 1-7 in the manufacture of a medicament, especially an oral or topical medicament for treating skin diseases caused by the JAK pathway, especially alopecia areata diseases.
